# EUROPEAN PATENT SPECIFICATION

(11) **EP 0 667 894 B1**
(45) Date of publication and mention of the grant of the patent: **02.01.1997**
(21) Application number: 92923696.6
(22) Date of filing: 07.10.1992
(51) Int. Cl.: C12M 1/14

(54) **A CELL CULTIVATING DEVICE**
VORRICHTUNG ZUR KULTIVIERUNG VON ZELLEN
DISPOSITIF CONCU POUR LA CULTURE DE CELLULES

(30) Priority: 15.11.1991 EP 91610090
(43) Date of publication of application: 23.08.1995
(73) Proprietor: NUNC A/S, DK-4000 Roskilde (DK)
(72) Inventor: JOHANSSON, Arne, DK-4320 Lejre (DK); ESSER, Peter, DK-2300 Kobenhavn S (DK)
(74) Representative: Plougmann, Vingtoft & Partners A/S
(86) International application number: DK9200295
(87) International publication number: WO9310211

(56) References cited:
- FR-A- 841 569
- GB-A- 1 539 263
- US-A- 4 927 764

## Description

The present invention relates to a cell cultivating device.

Developments within cell biology have led to increased demands for devices for the production of eucariotic cells, with the purpose of producing biologically active compounds produced by the cells.

For static cultures it is well known to use a flat, flask- or bottle-like, closed container having a neck defining an opening which may be closed by means of a screw cap. When the container is in use it is arranged with a pair of spaced, parallel larger side walls in a substantially horizontal position, and a liquid cell cultivating medium covers the inner surface of the lower container wall, which may have been subject to a surface treatment allowing good cell attachment. As cell cultivation may take place only on the inner surface of the lower container wall or the bottom wall the production capacity of such a flask- or bottle-like container is rather limited.

US-A-4,734,373 discloses a disposable cell culture device comprising a flask-like container with a neck defining an opening for introducing and removing cultivating medium and cells. The container contains a micro-constructed embodiment integrated in a unitary structure with the bottom wall of the container. The micro-embodiment comprises mutually spaced, interconnected plates having pores defined therein. When in use the micro-embodiment is submerged into a cultivating medium contained in the container. After use the cultivating medium may be discharged through the container neck, and the cells produced may be flushed out from the container in a known manner.

DK-B-143568 discloses an apparatus for mass cultivation of diploid cells, the apparatus being formed by a stack of superposed trays clamped together by means of bolts so as to define superposed closed cultivating chambers, which are interconnected by inlet passages. When a suitable total amount of cultivating medium has been filled into the apparatus through the inlet passages this medium may be distributed substantially evenly in the various chambers by placing the apparatus so that the chambers extend vertically, and subsequently moving the apparatus back to its normal position in which the chambers are horizontal. The upper surface of each tray may have been subject to a treatment securing improved cell attachment and growth.

The latter known cultivating device or apparatus may comprise any desired number of trays so that a large area covered with liquid cultivating medium may be obtained. However, care must be taken to ensure that adjacent trays are kept in sealing engagement with each other in order that cultivating medium from the cultivating chambers cannot leak out between the adjacent trays defining the respective chamber, and so as to prevent gas from escaping from and prevent atmospheric air from flowing into the cultivating chambers.

GB-A-1,539,263 discloses an apparatus for growing cells and comprising a plurality of cell attachment plates, which are arranged one above the other, and which are contained within a closed container having a filling or inlet opening for cell suspension. Each cell attachment plate, which is rectangular, is fused with the adjacent container walls along three sides. At one side each cell attachment plate is provided with an upstanding wall, which is spaced from the adjacent container wall.

In order to fill cell suspension into this known apparatus and have it distributed on the various cell attachment plates, the apparatus is placed in a filling position in which the apparatus is supported by one of the container side walls. Thereafter, the apparatus is tilted to a second position in which the apparatus is supported by another container side wall, and finally, the apparatus is tilted to a position of use in which the cell attachment plates extend horizontally and the apparatus is supported by the container bottom wall or by feet provided thereon.

The present invention provides an improved cell cultivating device which is more simple and convenient to use.

The present invention provides a cell cultivating device or apparatus comprising a container having a substantially flat bottom wall, a top wall, a substantially flat first end wall extending at substantially right angles to the flat bottom wall, a second end wall, and opposite side walls interconnecting the top and bottom walls and the end walls, a closeable filling opening being defined in one of said walls, the inner space of the container being divided into superposed cultivating chambers by at least one flat partition wall forming a chamber bottom wall and extending substantially parallel with the flat container bottom wall, all of the cultivating chambers being mutually interconnected so that liquid introduced into the container via the closeable filling opening may distribute itself in all of the interconnected chambers when the container is placed in an upright position in which it is supported by the flat first end wall, and the cell cultivating device according to the invention is characterized in that the superposed cultivating chambers are interconnected by an interconnecting passage defined at. an inner side surface part of the flat first end wall, each chamber communicating with the interconnecting passage through a connecting opening extending along only a small fraction of the length of the first end wall and being spaced from the bottom wall of the respective cultivating chamber.

A cell suspension or a cultivating liquid may be introduced into the container through the filling opening while the cell cultivating device is in a position in which it is supported by the flat first end wall of the container. In this position the liquid introduced into the container may distribute itself in the various cultivating chambers which are mutually interconnected by the interconnecting passage. When the liquid has distributed itself within the chambers the cell cultivating device may immediately be turned into a position in which it is supported by the flat bottom wall of the container and in which the partition walls or cell attachment walls are substantially horizontal. As the connecting openings are relatively short no substantial amount of the liquid distributed in the cultivating chambers may flow from one chamber into another when the cell cultivating device is suddenly turned from its filling position into its position of use in which it is supported by the bottom wall of the container.

The edges of the flat partition walls or cell attachment walls may be fused or sealed with the adjacent side walls and end walls of the container. In the preferred embodiment, however, each partition wall is a tray-like member having a peripheral wall encircling the partition wall and engaging with adjacent inner surface parts of the container walls, said peripheral wall extending from the partition wall towards the top wall of the container.

The liquid cultivating medium may be introduced into the superposed cultivating chambers so that the upper surface of each of the tray-like members and possibly also the inner surface of the bottom wall of the outer container may be covered by the liquid cell cultivating medium. This means that while the advantages of a closed container are maintained the cell cultivating area may be multiplied as desired by choosing a corresponding number of tray-like members.

The cell cultivating device according to the invention may be used for the production of various biological substances, such as viruses, vaccines, interferon, interleukin, hormones, antibodies, etc. Most cells used for the manufacture of the products mentioned above grow better and often only when attached to a suitable substratum. Therefore, at least part of the upper surfaces of the tray-like members and possibly also of the inner surface of the bottom wall of the outer container may be treated in a manner which secures optimum cell attachment and growth.

The outer container and/or the tray-like members may be made from metal, glass or plastics, preferably from polystyrene.

The tray-like members may, for example, be supported by inwardly directed projections or shelves formed on the inner side surfaces of side walls of the outer container. In the preferred embodiment, however, each tray-like member is arranged on top of and supported by a free edge portion of the peripheral wall of an adjacent lower tray-like member. The lowermost tray-like member may then be supported, for example by adjacent wall parts of the outer container in spaced relationship with the bottom wall of the container so as to define a cultivating chamber between the lower tray-like member and the container bottom wall.

As mentioned above the tray-like members may be supported by projections formed on the inner surfaces of the side walls of the outer container, whereby the tray-like members may be mutually spaced as desired. However, the mutual spacing of the tray-like members may alternatively be obtained by spacing members or legs arranged on the bottom surface of the lowermost tray member and/or on the adjacent container wall. Such spacing members may, for example, be spacing members or ribs formed integrally with and extending from said container wall.

When the cultivating device comprises two or more tray-like members assembling of the various parts of the cultivating device is facilitated if the tray-like members are mutually interconnected, for example by spot welding, so as to form a unit which may in turn be attached to the inner surface of the outer container, if desired. Alternatively, the tray-like members may be removeably or detachably arranged within the closed outer container and even in this case leakage of cultivating medium to the environment and gas flows into and out from the inner space of the cultivation device are effectively prevented.

It has been found that in practical use the cultivating medium, which has been poured into the cultivating device, tends to foam, and that the foaming medium arranged within the superposed cultivating chambers tends to leak through capillary spaces defined between the upstanding peripheral wall of a tray-like member and adjacent surface parts of the outer container and/or an adjacent tray-like member. Therefore, part of the foaming cultivating medium contained in an upper cultivating chamber tends to leak downwardly into an underlying chamber. In order to prevent or substantially reduce such leakage adjoining tray-like members may be sealingly interconnected along a major part of the periphery thereof and the non-interconnected part of the periphery of the adjoining tray-like members may be substantially spaced so as to define openings therebetween, namely the connecting openings formed in the interconnecting passage, and other openings interconnecting the filling opening of the outer container and the various cultivating chambers. Thus, the tray-like members of the cell-cultivating device may be heat-sealed, glued or otherwise sealingly interconnected along their periphery so as to form a unitary insert.

The outer container may be made from at least two separate parts, which may be sealingly interconnected when the tray-like members have been arranged within the container. Like the tray-like members the container parts may be interconnected, for example by glueing, heat sealing, ultra-sonic sealing or by any other suitable sealing method.

The liquid cell cultivating medium may be introduced into the cultivating device through the filling opening which may be sealingly closed by means of a closing member of any suitable type, such as a screw cap. The filling opening may in principle be formed in any of the top and bottom walls or side walls of the container. However, the closeable filling opening is preferably formed in said second end wall of the container, and the liquid cultivating medium may then conveniently be poured into the container while it is supported by its flat first end wall, in which position the liquid poured into the container may distribute itself into the various cultivating chambers via the interconnecting passage.

The interconnecting passage may be a tunnel defined between an inner side surface part of the flat first end wall and an opposite outer surface part of the peripheral wall of each tray-like member. Such tunnel may be formed by a channel defined by inner surface parts of the flat container defined in a strengthening rib of the container end wall. Alternatively or additionally, the tunnel may be formed by a channel defined by said outer surface part of the peripheral wall.

The interconnecting passage or tunnel may have any cross-sectional shape, such as a circular, semi-circular, or triangular shape. In the preferred embodiment, however, the interconnecting passage has a quadrangular, such as a trapezoidal or a rectangular (including a square), cross-sectional shape.

The tunnel - which may be defined by aligned channels formed in outer surface parts of the peripheral walls of the superposed tray-like members and/or adjacent container end wall and arranged in end to end relationship - interconnects the various superposed cultivating chambers. Thus, the tunnel may have open ends positioned opposite to and spaced from the bottom and top walls, respectively, of the container, whereby the upper and lower cultivating chambers are interconnected. Each intermediate cultivating chamber may be connected with the tunnel via a connecting opening, which is defined in a tunnel wall part spaced from the inner surface part of the adjacent flat first end wall. Each connecting opening is preferably positioned adjacent to the partition wall forming a chamber top wall of the respective cultivating chamber.

When a liquid cultivating medium or cell suspension has been poured into the container while it is in its filling position and supported by its flat first end wall and the liquid has been allowed to distribute itself within the various chambers via the interconnecting passage, the cell cultivating device may be tilted directly to its position of use in which it is supported by the flat bottom wall of the container, and in which all of the substantially parallel partition walls extend substantially horizontally. As the end openings and the connecting openings of the tunnel or interconnecting passage are relatively small, only rather small amounts of liquid can escape from one cultivating chamber to another during such tilting movement. As an example, each connecting opening may extend along less than 1/3 and preferably less than 1/4 of the length of the first end wall of the container. As an example, the connecting opening may extend along only approximately 1/10 of the length of the first end wall, whereby the amount of liquid which may move from one chamber to another by tilting the cultivating device from its filling position to its position of use in which it is supported by the bottom wall of the container will be negligible.

As is it desireable to utilize the inner volume of the outer container of the cultivating device to the highest possible extent the peripheral wall of each tray-like member should preferably be in abutting engagement with the opposite container end walls and container side walls along at least the major part of the outline of the container. This means that no substantial amount of liquid can flow from one chamber to another through the space defined between the peripheral wall of the tray-like member and the adjacent inner wall surface of the container when the cell cultivating device is tilted from its filling position to its position of use.

As mentioned above, a cell cultivating medium or cell suspension poured into the cell cultivating device may tend to foam, and the foaming liquid may tend to leak from an upper to a lower cultivating chamber via the capillary space defined between the peripheral wall of each tray-like member and the adjacent inner wall surface of the container. In order to counteract such tendency of leakage from the upper tray-like member the free edge of the peripheral wall of the tray-like member positioned adjacent to the top wall of the container may be spaced from this top wall. Such spacing may, for example, be 1-2 mm or even more.

The leakage of liquid from the upper tray-like member may alternatively or additionally be counteracted by forming the free edge of the peripheral wall of the tray-like member positioned adjacent to the top wall of the container so that it defines a sharp edge. Alternatively or additionally, this free edge of the peripheral wall may be made hydrophobic, for example by applying a layer of hydrophobic material, such as silicone, to such free edge.

When cells have been cultivated within the cultivating device according to the invention they may be scraped from the tray-like members, for example by means of a scraping implement being inserted through the filling or liquid inlet opening formed in the container, or the cells may be flushed out in any conventional manner. It is also possible to cut a wall part from the container so that the tray-like members, which may be interconnected to a unit or mutually separate, may be removed from the outer container prior to scraping the cultivated cells from the tray-like members. Alternatively, the container may comprise a tear-off or peel-off wall part. Such tear-off wall part may, for example, be made from a metal foil or from a plastic film which has been fastened to the remaining part of the container, for example by heat sealing or glueing. In an alternative embodiment, however, the tear-off wall is defined by a score line or weakening line formed in a container wall.

The container may then be formed by an upwardly open container part comprising a bottom wall and peripheral walls extending upwardly therefrom and having an upper rim portion and by a flat tear-off wall part, for example made from metal foil or plastic film, forming the top wall of the container. When the tray-like members have been inserted into the upwardly open container part the tear-off part may be detachably sealed to the upper rim portion of the upwardly open container part in any suitable manner, such as by glueing or heat sealing. Alternatively, the tear-off wall part may form an end wall of the container.

The closeable filling opening of the container may open into a manifold chamber defined within the container and communicating with at least one of the cultivating chambers via an inlet opening formed in at least one of the tray-like members.

The inlet openings may be overflow openings formed in the tray-like members, for example notches or cut-outs formed in the upper edge portions of the peripheral walls of the tray-like members. Such notches or cut-outs may possibly extend substantially the total length of the respective peripheral wall. Provided that the liquid level in each tray member does not reach the overflow opening, the liquid cannot escape from the tray member through the inlet opening when the cell cultivating device is supported by the bottom wall of the container and is in its position of use.

The outer closed container may be of any suitable size and shape, and the separate tray-like members arranged therein may have a corresponding contour. As an example, the outer container may be a conventional tissue culture flask providing a relatively small cell growth area at the inner surface of the bottom wall of the flask, for example in the order of 25-175 cm². Such a flask may, for example, be provided with two or three tray-like members, whereby the growth area may be increased by a factor three or four, respectively.

According to a further aspect the present invention provides a cell cultivating device or apparatus comprising a container having a substantially flat bottom wall, a top wall, a substantially flat first end wall extending at substantially right angles to the flat bottom wall, a second end wall, and opposite side walls interconnecting the top and bottom walls and the end walls, a closeable filling opening being defined in one of said walls, the inner space of the container being divided into cultivating chambers by at least two superposed tray-like members each having a flat partition wall and an encircling peripheral wall extending from the partition wall towards the top wall of the container and engaging with adjacent inner surface parts of the container walls, the partition walls, which are mutually spaced and spaced from the top and bottom walls, respectively, of the container extending substantially parallel with the bottom wall of the container, all of the cultivating chambers being mutually interconnected so that liquid introduced into the container via the closeable filling opening may distribute itself in all of the interconnected chambers when the container is placed in an upright position in which it is supported by the flat first end wall, and the cell cultivating device according to the invention is characterized in that adjoining tray-like members are sealingly interconnected along a major part of the periphery thereof, the tray-like members being spaced along remaining, non-interconnected parts of said periphery.

The invention will now be further described with reference to the drawings, wherein
Figs. 1 and 2 are perspective views of a first embodiment of the cultivating device according to the invention,
Figs. 3 and 4 are perspective views of a second embodiment,
Figs. 5 and 6 are perspective views showing a third embodiment of the cultivating device according to the invention,
Figs. 7 and 8 are detailed perspective views showing a passage for interconnecting superposed cultivating chambers and formed in an upper and lower tray-like member, respectively, and
Figs. 9 and 10 illustrate various embodiments of the upper edge portion of the tray-like members.

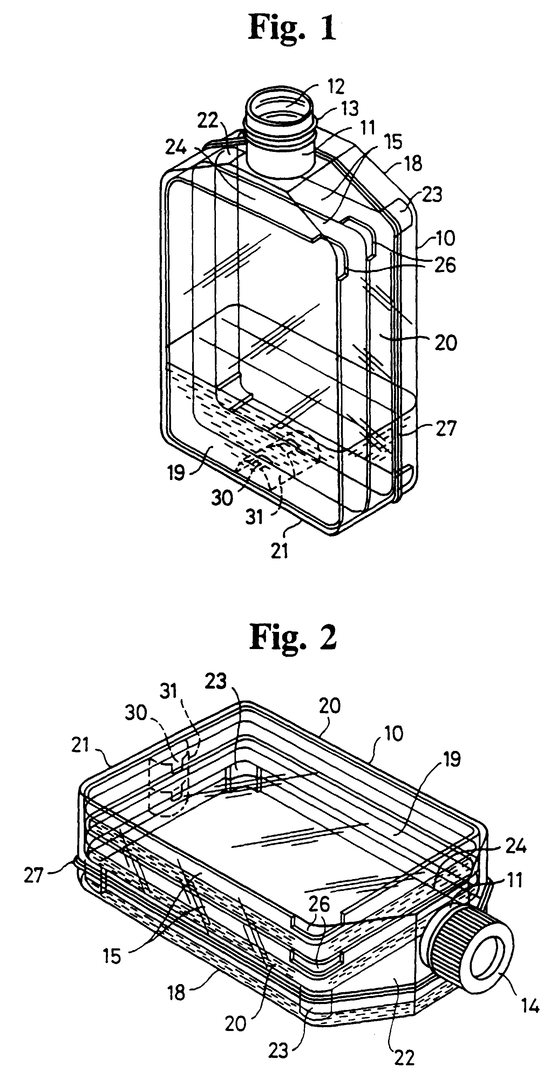

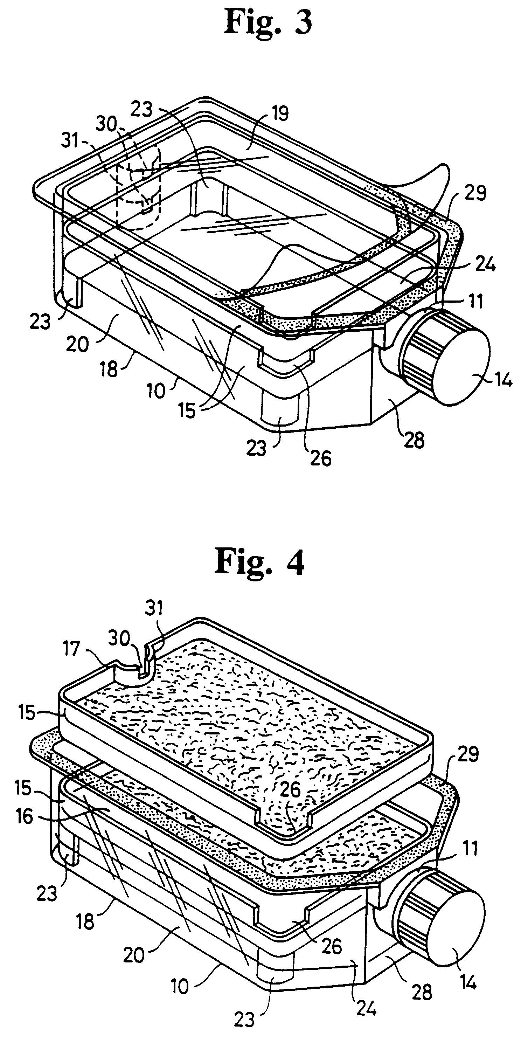

The cell cultivating device or apparatus shown in Figs. 1 and 2 comprises an outer container or flask 10, which is made from a transparent or intransparent material, such as glass or plastics material, or from metal. The container 10 is provided with a neck 11 defining a filling opening 12. The neck 11 is formed with outer screw threads 13 for cooperating with inner screw threads of a screw cap 14 by means of which the filling opening 12 may be closed. A number of tray members 15 having a bottom wall or cell attachment plate 16 and an encircling peripheral side wall 17 (Fig. 4) extending upwardly therefrom, is arranged in superposed relationship within the container 10, so as to define cultivating chambers therein. The outer container 10 has a flat bottom wall 18, a top wall 19, opposite side walls 20, a flat end wall 21, and an opposite end wall 22 on which the neck 11 is formed.

The lower tray member arranged adjacent to the bottom wall 18 is supported by feet or spacer members 23 so that a cultivating chamber is defined between the inner surface of the container bottom wall 18 and the lower surface of the bottom wall 16 of the lower tray member 15. The spacer members 23 may be formed as integral parts of the walls of the outer container 10 or of the lower tray member. An upper cultivating chamber is defined between the inner surface of the container top wall 19 and the upper surface of the adjacent bottom wall 16 of the upper tray member 15. Furthermore, a cultivating chamber is defined between each pair of adjacent tray members 15. Thus, the embodiment shown in Figs. 1 and 2 which comprises two tray members 15 defines three cultivating chambers therein.

The container end wall 22 has a convex contour so that a manifold chamber 24 is defined between the container end wall 22 and the adjacent side walls 17 of the tray members 15. Cut-outs in the peripheral side walls 17 of the tray members 15 define overflow openings 26 communicating with the manifold chamber 24.

The outer container 10 may be made from at least to separate container parts, which may, for example, be moulded from transparent plastics material. When the tray members 15 have been arranged within one of the container parts, such container parts may be sealingly interconnected, for example by interconnecting flanged rim portions of the container parts so as to form a heat seal 27 in a plane being substantially parallel with the bottom walls 16 of the tray members 15.

As indicated in broken lines in Figs. 1 and 2 each of the tray-members 15 further comprises an equalizing opening 30 formed in its peripheral side wall 17 adjacent to the container end wall 21. The equalizing opening 30 may be a cut-out formed in the rim portion of a curved part of the peripheral wall 17 of each tray-member. The curved parts of the superposed tray-members 15 define a transverse tunnel or passage 31 interconnecting the superposed cell cultivating chambers. The tunnel or passage 31 may have any suitable cross-sectional shape, which may, for example, be a semi-circular as shown.

When the cell cultivating device shown in Figs. 1 and 2 is to be used it is positioned in its upright position shown in Fig. 1, and the screw closure cap 14 is removed. A suitable amount of cell cultivating medium and cells to be cultivated are poured into the manifold chamber 24 of the container 10 through the filling opening 12 defined by the neck 11. From the manifold chamber 24 the liquid cultivating medium flows into one or more of the various cultivating chambers via the overflow opening 26 and through the opening defined between the inner surface of the container bottom wall 18 and the bottom wall 16 of the adjacent tray member 15. Now, as the cultivating chambers are all interconnected by the tunnel 31 and the openings 30 formed therein by the principle that liquid seeks its own level the liquid level within the cultivating chambers will be the same after a short period of time. Provided that the dimensions of the cultivating chambers are substantially the same, these chambers will now contain substantially the same amount of liquid.

When the filling opening 12 has been closed by means of the screw cap 14 the container or flask 10 may by a quick movement be tilted from the upright position shown in Fig. 1 to the position shown in Fig. 2 in which the container bottom wall 18 is supported in a substantially horizontal position. As the equalizing opening are relatively small, this simple procedure renders it possible to have substantially the same amount of liquid cultivating medium placed in all of the now horizontally extending cultivating chambers. This means that a layer of liquid cultivating medium containing cells to be cultivated is supported by the container bottom wall 18 and by the bottom walls or cell attachment plates 16 of each of the tray members 15. The surfaces of these walls have preferably been subject to a surface treatment allowing good cell attachment.

After expiration of the cultivating period the liquid cultivating medium may be poured out through the inlet opening 12 of the neck 11. Thereafter, the cells attached to the bottom walls 16 and 18 may be scraped or flushed out through the inlet or filling opening 12 in a manner known per se. Alternatively, the container bottom wall 18 or top wall 19, or any of the container side or end walls 20 and 21, respectively, may be cut away or otherwise removed so that the tray members may be taken out from the container 10, whereafter the cultivated cells may be scraped flushed from the bottom walls 16 and 18.

The embodiments shown in Figs. 3-8 are modified embodiments of the embodiment shown in Figs. 1 and 2, and similar parts have been provided with same reference numerals.

The embodiment shown in Figs. 3 and 4 differs from that shown in Figs. 1 and 2 in that the container top wall 19, which may be made from a flexible plastic film, is removably connected to a more stiff container main part 28. When the tray members 15 have been arranged within the upwardly open container main part 28 the top wall 19 may be sealingly and removably connected to a substantially plane flange portion 29 by heat sealing or by means of an adhesive.

It should be understood that the tear-off wall could alternatively be defined by weakening lines or score lines formed in a wall part of the outer container 10, for example in the top wall 19. Even when the top wall has not be provided with such score lines, the main part of the top wall 19 may be cut out by means of a suitable cutting tool so as to form an opening through which the tray members 15 may be taken out from the outer container 10.

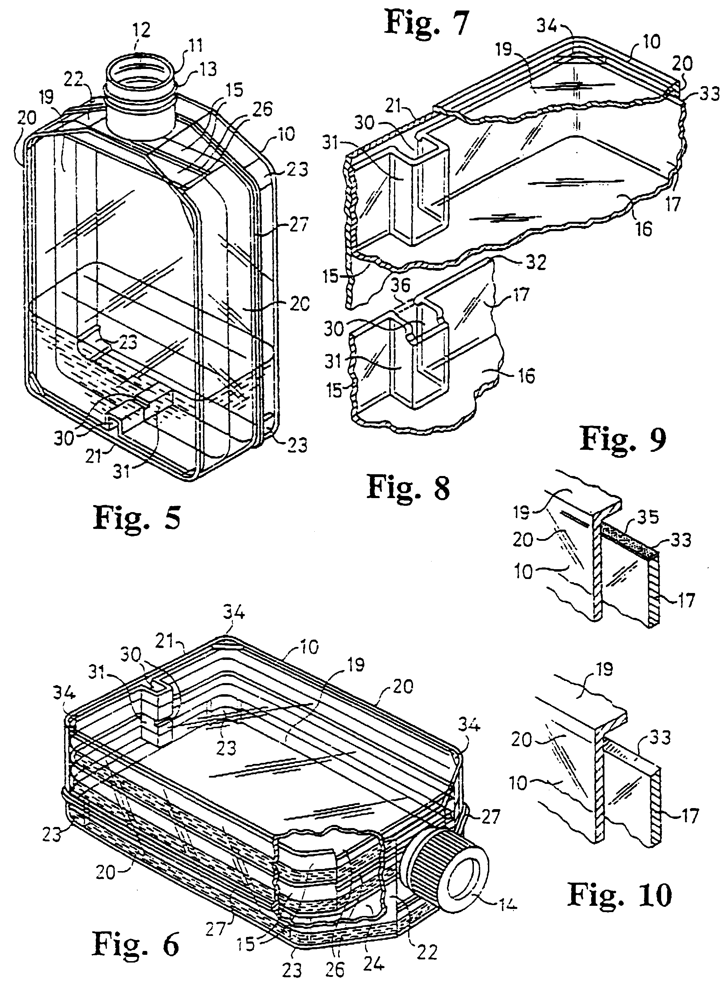

In Figs. 5-8 the interconnecting passage or tunnel, which is defined by aligned outer channels in the upstanding peripheral side walls 17 of the tray members 15, has a rectangular or square cross-sectional shape. The cultivating device or apparatus comprises two superposed tray-like members 15 defining three cultivating chambers within the container or flask 10. In order to prevent foaming liquid in any of the tray-like members to leak into a lower cultivating chamber or compartment, the upper free edge 32 (Fig. 8) of the lower tray-like member 15 is sealingly connected to the adjacent bottom surface of the upper tray-like member, for example by heat sealing or gluening. The tray-like members 15 are preferably sealingly interconnected along their total outline, except for the openings 26 and 30.

In order to reduce or eliminate leakage of foaming liquid from the upper tray-like member 15 into one of the lower chambers or compartments via the capillary space defined between the peripheral side wall 17 of the upper tray-like member and the adjacent inner surface of the container walls, the upper free edge 33 (Figs. 7, 9 and 10) is spaced from the inner surface of the top wall 19, for example by means of spacer lugs 34 formed on the inner side surface of the top wall 19 at the corners thereof. The spacing of the upper free edge 33 of the peripheral wall 17 from the inner surface of the top wall 19 may, for example, be about 1-2 mm.

Alternatively or additionally, the upper free edge of the side wall 17 of the upper tray-like member may be provided with a layer 35 of a hydrophobic material as illustrated in Fig. 9 and/or the upper free edge 33 may be sharpened so as to define a sharp edge as illustrated in Fig. 10. As the free edge 33 of the upper tray-like member is spaced from the inner surface of the top wall 19 the tunnel 31 may interconnect the upper and lower cultivating chambers or compartments via its open ends, which means that no cut-out is needed in the tunnel walls at its upper end as shown in Figs. 2 and 4. The intermediate cultivating chamber or compartment may, however, be in communication with the inner space of the tunnel 31 through a cut-out 30 in the tunnel wall at the upper edge of the lower tray-like member. This cut-out is preferably spaced inwardly from the plane of the adjacent peripheral wall 17 as best illustrated in Fig. 8.

As indicated by dotted lines in Fig. 8, each of the outer channels formed in the peripheral side walls 17 of the tray-like members 15 may be closed by an outer wall part 36. This means that the tunnel 31 is defined totally by wall parts of the tray-like members 15.

In the embodiments illustrated in Figs. 1-4, the superposed tray-like members could be sealingly interconnected so as to form a unitary insert as explained in connection with Figs. 5-8. Similarly, in Figs. 1-4 the upper free edge of the upper tray-like member 15 may be spaced from the inner surface of the container top wall 19. Furthermore, the interconnecting passage or tunnel 31 shown in the drawings could be replaced by two or more such tunnels each having a smaller cross-sectional area. As an example, the interconnecting passage or passages could be defined in rib-like depressions formed in the flat end wall 21 of the container.

## Claims

1. A cell cultivating device comprising a container (10) having a substantially flat bottom wall (18), a top wall (19), a substantially flat first end wall (21) extending at substantially right angles to the flat bottom wall (22), a second end wall, and opposite side walls (20) interconnecting the top and bottom walls and the end walls, a closeable filling opening (12) being defined in one (22) of said walls, the inner space of the container being divided into cultivating chambers by at least first and second superposed tray-like members (15) each having a flat partition wall (16) and an encircling peripheral wall (17) extending from the partition wall towards the top wall (19) of the container (10) and engaging with adjacent inner surface parts of the container walls, the partition walls (16), which are mutually spaced and spaced from the top and bottom walls (18, 19), respectively, of the container (10) extending substantially parallel with the bottom wall of the container, all of the cultivating chambers being mutually interconnected so that liquid introduced into the container via the closeable filling opening (12) may distribute itself in all of the interconnected chambers when the container is placed in an upright position in which it is supported by the flat first end wall (21), characterized in that the second tray-like member (15) is supported by a free edge portion of the peripheral wall (17) of the first tray-like member (15) and in that the superposed cultivating chambers are interconnected by an interconnecting passage (31) defined at an inner side surface part of the flat first end wall (21), each cultivating chamber communicating with the interconnecting passage through a connecting opening (30) extending along only a small fraction of the length of the first end wall (21) and being spaced from the bottom wall (16, 18) of the respective cultivating chamber.

2. A cell cultivating device according to claim 1, wherein the partition wall (16) of the first tray-like member (15) is separated from the bottom wall (18) of the container (10) so as to define a cultivating chamber between the first tray-like member and the container bottom wall.

3. A cell cultivating device according to claim 1 or 2, wherein adjoining tray-like members (15) are sealingly interconnected along a major part of the periphery thereof and spaced along a remaining, non-interconnected part of said periphery.

4. A cell cultivating device according to any of the claims 1-3, wherein the closeable filling opening (12) is formed in said second end wall (22) of the container (10).

5. A cell cultivating device according to any of the claims 1-4, wherein the interconnecting passage is a tunnel (31) defined between said inner side surface part of the flat first end wall (21) and an opposite outer surface part of the peripheral wall (17) of each tray-like member (15).

6. A cell cultivating device according to claim 5, wherein said outer surface part of the peripheral wall (17) defines a channel.

7. A cell cultivating device according to any of the claims 1-4, wherein the interconnecting passage is a tunnel (31) extending through and being defined totally by the peripheral wall (17) of each tray-like member (15).

8. A cell cultivating device according to any of the claims 1-7, wherein the interconnecting passage (31) has a quadrangular cross-sectional shape.

9. A cell cultivating device according to claim 8, wherein the interconnecting passage (31) has a substantially rectangular cross-sectional shape.

10. A cell cultivating device according to any of the claims 5-9, wherein the tunnel (31) forming the interconnecting passage has open ends positioned opposite to and spaced from the bottom and top walls (18, 19), respectively, of the container (10), a connecting opening (30) connecting a cultivating chamber defined between adjacent partition walls (16) with the interconnecting passage (31) and being defined in a tunnel wall part, which is spaced from the inner surface part of the flat first end wall (21).

11. A cell cultivating device according to any of the claims 1-10, wherein each connecting opening (30) extends along less than 1/3 and preferably less than 1/4 of the length of the first end wall (21) of the container (10).

12. A cell cultivating device according to claim 11, wherein the connecting opening (30) extends along approximately 1/10 of the length of the first end wall (21).

13. A cell cultivating device according to any of the claims 1-12, wherein except for the interconnecting passage (31) the peripheral wall (17) of each tray-like member (15) is in abutting engagement with adjacent inner surface parts of the flat first end wall (21) and of adjacent parts of the opposite side walls (20) of the container (10).

14. A cell cultivating device according to any of the claims 1-13, wherein the free edge of the peripheral wall of the tray-like member positioned adjacent to the top wall of the container is spaced from said top wall.

15. A cell cultivating device according to claim 14, wherein said spacing is 1-2 mm.

16. A cell cultivating device according to any of the claims 1-15, wherein the free edge (33) of the peripheral wall (17) of the tray-like member (15) positioned adjacent to the top wall (19) of the container (10) defines a sharp edge.

17. A cell cultivating device according to any of the claims 1-16, wherein the free edge (33) of the peripheral wall (17) of the tray-like member (15) positioned adjacent to the top wall (19) of the container (10) is hydrophobic.

18. A cell cultivating device according to any of the claims 1-17, wherein the tray-like members (15) define an insert arranged within the container (10).

19. A cell cultivating device according to claim 18, wherein the container (10) is made from two parts which have been sealingly interconnected after insertion of said insert therein.

20. A cell cultivating device according to any of the claims 1-19, wherein the container (10) comprises a tear-off wall part (19).

21. A cell cultivating device according to claims 20 and 21, wherein the tear-off wall part (19) is one of said container parts.

22. A cell cultivating device according to any of the claims 1-21, wherein the closeable filling opening (12) communicates with a manifold chamber (24) defined within the container (10) and being in communication with at least one of the cultivating chambers via an inlet opening (26) formed in the peripheral wall (17) of at least one of the tray-like members (15).

23. A cell cultivating device according to any of the claims 1-22, wherein the container (10) is a bottle or flask.

## Patentansprüche

1. Zellzuchtvorrichtung mit einem Behälter (10) mit einer im wesentlichen ebenen Bodenwand (18), einer oberen Wand (19), einer sich im wesentlichen rechtwinklig zu der ebenen Bodenwand erstreckenden im wesentlichen ebenen ersten Abschlußwand (21), einer zweiten Abschlußwand (22) und die obere und die Bodenwand sowie die Abschlußwände verbindenden gegenüberliegenden Seitenwänden (20), wobei eine verschließbare Einfüllöffnung (12) in einer (22) der Wände festgelegt ist, der Innenraum des Behälters durch zumindest ein erstes und ein zweites übereinanderliegendes schalenartiges Element (15) mit jeweils einer ebenen Trennwand (16) und einer sich von der Trennwand auf die obere Wand (19) des Behälters (10) zu erstreckenden und mit benachbarten Innenflächenteilen der Behälterwände in Eingriff stehenden umlaufenden Außenwand (17) in Zuchtkammern geteilt ist, die Trennwände, die voneinander und von der oberen bzw. der Bodenwand (18, 19) des Behälters (10) in einen Abstand gesetzt sind, sich im wesentlichen parallel zu der Bodenwand des Behälters erstrecken, alle Zuchtkammern miteinander verbunden sind, so daß sich durch die verschließbare Einfüllöffnung (12) eingeführte Flüssigkeit auf alle verbundenen Kammern verteilen kann, wenn der Behälter in eine aufrechte Lage gestellt wird, in der er von der ebenen ersten Abschlußwand (21) getragen wird, dadurch **gekennzeichnet**, daß das zweite schalenartige Element (15) von einem Bereich einer freien Ecke der Außenwand (17) des ersten schalenartigen Elements (15) getragen ist und dadurch daß die übereinanderliegenden Zuchtkammern durch einen an einem Innenflächenteil der ebenen ersten Abschlußwand (21) festgelegten verbindenden Durchgang (31) verbunden sind, wobei jede Zuchtkammer durch eine sich entlang nur einem kleinen Teil der Länge der ersten Abschlußwand (21) erstreckenden und von der Bodenwand (16, 18) der entsprechenden Zuchtkammer in einen Abstand gesetzten verbindenden Öffnung (30) mit dem verbindenden Durchgang in Verbindung steht.

2. Zellzuchtvorrichtung nach Anspruch 1, bei der die Trennwand (16) des ersten schalenartigen Elements (15) von der Bodenwand (18) des Behälters (10) entfernt ist, so daß zwischen dem ersten schalenartigen Element und der Behälterbodenwand eine Zuchtkammer festgelegt ist.

3. Zellzuchtvorrichtung nach Anspruch 1 oder 2, bei der benachbarte schalenartige Elemente (15) entlang einem größeren Teil ihres Umfangs dicht miteinander verbunden sind und entlang einem verbleibenden, nicht verbundenen Teil des Umfangs in einen Abstand gesetzt sind.

4. Zellzuchtvorrichtung nach einem der Ansprüche 1-3, bei der die verschließbare Einfüllöffnung (12) in der zweiten Abschlußwand (22) des Behälters (10) ausgebildet ist.

5. Zellzuchtvorrichtung nach einem der Ansprüche 1-4, bei der der verbindende Durchgang ein zwischen dem genannten Innenflächenteil der ebenen ersten Abschlußwand (21) und einem gegenüberliegenden Außenflächenteil der Außenwand (17) jedes schalenartigen Elements (15) festgelegter Tunnel (31) ist.

6. Zellzuchtvorrichtung nach Anspruch 5, bei der der genannte Außenflächenteil der Außenwand (17) einen Kanal festlegt.

7. Zellzuchtvorrichtung nach einem der Ansprüche 1-4, bei der der verbindende Durchgang ein sich durch die Außenwand (17) jedes schalenartigen Elements (15) erstreckender und von dieser vollständig festgelegter Tunnel (31) ist.

8. Zellzuchtvorrichtung nach einem der Ansprüche 1-7, bei der der verbindende Durchgang (31) eine viereckige Querschnittsform hat.

9. Zellzuchtvorrichtung nach Anspruch 8, bei der der verbindende Durchgang (31) eine im wesentlichen rechteckige Querschnittsform hat.

10. Zellzuchtvorrichtung nach einem der Ansprüche 5-9, bei der der den verbindenden Durchgang bildende Tunnel (31) der Boden- bzw. oberen Wand (18, 19) des Behälters (10) gegenüberliegend und in einem Abstand davon angeordnete offene Enden hat, wobei eine verbindende Öffnung (30) eine zwischen benachbarten Trennwänden (16) festgelegte Zuchtkammer mit dem verbindenden Durchgang (31) verbindet und in einem Tunnelwandteil festgelegt ist, der von dem genannten Innenflächenteil der ebenen ersten Abschlußwand (21) in einen Abstand gesetzt ist.

11. Zellzuchtvorrichtung nach einem der Ansprüche 1-10, bei der sich jede verbindende Öffnung (30) entlang weniger als 1/3 und vorzugsweise weniger als 1/4 der Länge der ersten Abschlußwand (21) des Behälters (10) erstreckt.

12. Zellzuchtvorrichtung nach Anspruch 11, bei der sich die verbindende Öffnung (30) entlang ungefähr 1/10 der Länge der ersten Abschlußwand (21) erstreckt.

13. Zellzuchtvorrichtung nach einem der Ansprüche 1-12, bei der die Außenwand (17) jedes schalenartigen Elements (15), ausgenommen der verbindende Durchgang (31), in anliegendem Eingriff mit anschließenden Innenflächenteilen der ebenen ersten Abschlußwand (21) und der benachbarten Teile der gegenüberliegenden Seitenwände (20) des Behälters (10) ist.

14. Zellzuchtvorrichtung nach einem der Ansprüche 1-13, bei der die freie Ecke der Außenwand des anschließend an die obere Wand des Behälters angeordneten schalenartigen Elements in einen Abstand von der oberen Wand gesetzt ist.

15. Zellzuchtvorrichtung nach Anspruch 14, bei der der genannte Abstand 1-2 mm beträgt.

16. Zellzuchtvorrichtung nach einem der Ansprüche 1-15, bei der die freie Ecke (33) der Außenwand (17) des benachbart der oberen Wand (19) des Behälters (10) angeordneten schalenartigen Elements (15) eine scharfe Ecke festlegt.

17. Zellzuchtvorrichtung nach einem der Ansprüche 1-16, bei der die freie Ecke der Außenwand des benachbart der oberen Wand des Behälters angeordneten schalenartigen Elements hydrophob ist.

18. Zellzuchtvorrichtung nach einem der Ansprüche 1-17, bei der die schalenartigen Elemente einen innerhalb des Behälters angeordneten Einsatz darstellen.

19. Zellzuchtvorrichtung nach Anspruch 18, bei der der Behälter aus zwei Teilen hergestellt ist, die nach Einsetzen des Einsatzes darin dicht verbunden worden sind.

20. Zellzuchtvorrichtung nach einem der Ansprüche 1-19, bei der der Behälter ein Abreiß-Wandteil umfaßt.

21. Zellzuchtvorrichtung nach Anspruch 19 und 20, bei der der Abreiß-Wandteil (19) eines der Behälterteile ist.

22. Zellzuchtvorrichtung nach einem der Ansprüche 1-21, bei der die verschließbare Einfüllöffnung (12) mit einer Verteilerkammer (24) in Verbindung steht, die innerhalb des Behälters (10) festgelegt ist und durch eine in der Außenwand (17) zumindest eines der schalenartigen Elemente (15) ausgebildete Einlaßöffnung (26) mit zumindest einer der Zuchtkammern in Verbindung steht.

23. Zellzuchtvorrichtung nach einem der Ansprüche 1-22, bei der der Behälter (10) eine Flasche oder ein Kolben ist.

## Revendications

1. Un dispositif pour la culture de cellules comprenant un récipient (10) muni d'une paroi de fond sensiblement plane (18), d'une paroi de plafond (19), d'une première paroi d'extrémité (21) sensiblement plane s'étendant selon des angles sensiblement droits par rapport à la paroi de fond plane, d'une seconde paroi d'extrémité (22), et de parois latérales (20) opposées reliant les parois de plafond et de fond et les parois d'extrémité, une ouverture de remplissage (12) susceptible d'être obturée étant définie dans l'une desdites parois, l'espace intérieur du récipient étant divisé en chambres de culture par au moins un premier et un deuxième organes (15) superposés en forme de rayonnage, munis chacun d'une paroi de séparation plane (16) et d'une paroi périphérique (17) les entourant et s'étendant de la paroi de séparation vers la paroi de plafond (19) du récipient (10) et venant en contact avec les parties de surface intérieure adjacentes des parois du récipient, les parois de séparation (16) qui sont respectivement mutuellement espacées et espacées par rapport aux parois de plafond et de fond (18, 19) du récipient (10), s'étendant de façon sensiblement parallèle à la paroi de fond du récipient, toutes les chambres de culture étant mutuellement interconnectées de telle façon que du liquide introduit dans le récipient via l'ouverture de remplissage (12) susceptible d'être obturée puisse se distribuer de lui-même dans toutes les chambres interconnectées lorsque le récipient est placé dans une position verticale dans laquelle il est supporté par la première paroi plane d'extrémité (21), caractérisé en ce que le second organe en forme de rayonnage (15) est supporté par une partie de bord libre de la paroi périphérique (17) du premier organe en forme de rayonnage (15), en ce que les chambres de culture superposées sont interconnectées par un passage d'interconnexion (31) défini sur une partie de surface latérale intérieure de la première paroi d'extrémité plane (21), et en ce que chaque chambre de culture communique avec le passage d'interconnexion par une ouverture de liaison (30) s'étendant seulement le long d'une petite fraction de la longueur de la première paroi d'extrémité (21) et étant espacée de la paroi de fond (16, 18) correspondante de la chambre de culture.

2. Un dispositif pour la culture de cellules selon la revendication 1, dans lequel la paroi de séparation (16) du premier organe en forme de rayonnage (15) est séparée de la paroi de fond (18) du récipient (10) de façon à définir une chambre de culture entre le premier organe en forme de rayonnage et la paroi de fond du récipient.

3. Un dispositif pour la culture de cellules selon la revendication 2, dans lequel des organes adjacents en forme de rayonnage (15) sont interconnectés de façon étanche le long de la majeure partie de leur périphérie et sont espacés le long d'une partie non interconnectée restante de ladite périphérie.

4. Un dispositif pour la culture de cellules selon l'une quelconque des revendications 1 à 3, dans lequel l'ouverture de remplissage (12) susceptible d'être obturée est formée dans ladite seconde paroi d'extrémité (22) du récipient (10).

5. Un dispositif pour la culture de cellules selon l'une quelconque des revendications 1 à 4, dans lequel le passage d'interconnexion est un tunnel (31) défini entre ladite partie de surface latérale intérieure de la première paroi d'extrémité plane (21) et une partie de surface extérieure opposée de la paroi périphérique (17) de chaque organe en forme de rayonnage (15).

6. Un dispositif pour la culture de cellules selon la revendication 5, dans lequel ladite partie de surface extérieure de la paroi périphérique (17) définit un canal.

7. Un dispositif pour la culture de cellules selon l'une quelconque des revendications 1 à 4, dans lequel le passage d'interconnexion est un tunnel (31) s'étendant à travers la paroi périphérique et étant défini totalement par la paroi périphérique (17) de chaque organe (15) en forme de rayonnage.

8. Un dispositif pour la culture de cellules selon l'une quelconque des revendications 1 à 7, dans lequel le passage d'interconnexion (31) présente une forme quadrangulaire en section transversale.

9. Un dispositif pour la culture de cellules selon la des revendication 8, dans lequel le passage d'interconnexion (31) présente une forme sensiblement rectangulaire en section transversale.

10. Un dispositif pour la culture de cellules selon l'une quelconque des revendications 5 à 9, dans lequel le tunnel (31) formant le passage d'interconnexion comporte des extrémités ouvertes positionnées en opposition aux parois respectives de fond et de plafond (18, 19) et espacées de ces parois respectives du récipient (10), une ouverture de liaison (30) reliant une chambre de culture définie entre des parois de séparation (16) adjacentes et le passage d'interconnexion (31) et étant défini dans une partie de paroi de tunnel qui est espacée de la partie de surface intérieure de la première paroi d'extrémité plane (21).

11. Un dispositif pour la culture de cellules selon l'une quelconque des revendications 1 à 10, dans lequel chaque ouverture de liaison s'étend le long de moins d'1/3 et de préférence moins d'1/4 de la longueur de la première paroi d'extrémité (21) du récipient (10).

12. Un dispositif pour la culture de cellules selon la revendication 11, dans lequel l'ouverture de liaison (30) s'étend le long d'approximativement 1/10 de la longueur de la première paroi d'extrémité (21).

13. Un dispositif pour la culture de cellules selon l'une quelconque des revendications 1 à 12, dans lequel à l'exception du passage d'interconnexion (31), la paroi périphérique (17) de chaque organe en forme de rayonnage (15) est en contact de butée avec les parties de surface intérieure adjacentes de la première paroi d'extrémité plane (21) et les parties adjacentes des parois latérales (20) opposées du récipient (10).

14. Un dispositif pour la culture de cellules selon l'une quelconque des revendications 1 à 13, dans lequel le bord libre de la paroi périphérique de l'organe en forme de rayonnage positionné adjacent à la paroi de plafond du récipient est espacé de ladite paroi de plafond.

15. Un dispositif pour la culture de cellules selon la revendication 14, dans lequel ledit espacement est de 1 à 2 mm.

16. Un dispositif pour la culture de cellules selon l'une quelconque des revendications 1 à 15, dans lequel le bord libre (33) de la paroi périphérique (17) de l'organe en forme de rayonnage (15) positionné adjacent à la paroi de plafond (19) du récipient (10) définit une arête vive.

17. Un dispositif pour la culture de cellules selon l'une quelconque des revendications 1 à 16, dans lequel le bord libre de la paroi périphérique de l'organe en forme de rayonnage positionné adjacent à la paroi de plafond du récipient est hydrophobe.

18. Un dispositif pour la culture de cellules selon l'une quelconque des revendications 1 à 17, dans lequel les organes en forme de rayonnage définissent un insert disposé à l'intérieur du récipient.

19. Un dispositif pour la culture de cellules selon la revendication 18, dans lequel le récipient est réalisé en deux parties qui ont été interconnectées de façon étanche après l'insertion dudit insert dans leur intérieur.

20. Un dispositif pour la culture de cellules selon l'une quelconque des revendications 1 à 21, dans lequel le récipient comporte une partie de paroi détachable.

21. Un dispositif pour la culture de cellules selon les revendications 19 et 20, dans lequel la partie de paroi détachable (19) est l'une des parties dudit récipient.

22. Un dispositif pour la culture de cellules selon l'une quelconque des revendications 2 à 21, dans lequel l'ouverture de remplissage (12) susceptible d'être obturée communique avec une chambre de collecteur (24) définie à l'intérieur du récipient (10) et qui est en communication avec au moins l'une des chambres de culture via une ouverture d'entrée (26) formée dans la paroi périphérique (17) d'au moins l'un des organes en forme de rayonnage (15).

23. un dispositif pour la culture de cellules selon l'une quelconque des revendications 1 à 22, dans lequel le récipient (10) est une bouteille ou un flacon.
